# EUROPEAN PATENT SPECIFICATION

(11) **EP 2 921 138 B1**
(45) Date of publication and mention of the grant of the patent: **09.11.2022**
(21) Application number: 13854782.3
(22) Date of filing: 23.09.2013
(51) Int. Cl.: A61F 2/90, A61B 17/24, A61F 2/18

(54) **IMPLANT SYSTEM FOR TREATING SINUSITIS OR ALLERGIC RHINITIS**
IMPLANTATSYSTEM ZUR BEHANDLUNG VON SINUSITIS ODER ALLERGISCHER RHINITIS
SYSTÈME D'IMPLANT POUR LE TRAITEMENT DE LA SINUSITE OU DE LA RHINITE ALLERGIQUE

(30) Priority: 13.11.2012 CN 201210454911
(43) Date of publication of application: 23.09.2015
(73) Proprietor: Puyi (Shanghai) Biotechnology Co., Ltd, Pudong, Shanghai 201203 (CN)
(72) Inventor: XIE, Jian, Shanghai 201203 (CN); WEI, Zheng, Shanghai 201203 (CN)
(74) Representative: Wagner, Carsten
(86) International application number: PCT/CN2013/083986
(87) International publication number: WO 2014/075514

(56) References cited:
- WO-A1-2009/018248
- WO-A2-2006/107957
- WO-A2-2009/079418
- CN-A- 101 189 016
- CN-A- 102 512 272
- CN-A- 102 988 122
- CN-U- 201 565 010
- CN-Y- 2 526 020
- CN-Y- 2 824 975

## Description

### BACKGROUND OF THE INVENTION

### 1. Technical Filed

This invention relates generally to medical devices, and more particularly to an implanted system for treating sinusitis or allergic rhinitis.

### 2. Related Art

Sinusitis and allergic rhinitis are the common Ear-Nose-Throat (ENT) diseases for the person between 5 and 79 years old. Surveys of Health department show: currently, the global incidence of allergic rhinitis is up to 10-14%, and the incidence of sinusitis accounts for about 15% of the population, wherein nearly 600 million people are suffering from "harassment" of rhinitis, and the patient's number increases gradually in the whole world.

66% of asthma patients are victims of allergic rhinitis. According to experts, if patients of allergic rhinitis get an improper treatment, more than 1/3 of the patients eventually develop into the asthma patients.

Sinus is also known as paranasal sinus or accessory sinus, which includes a plurality of cavities containing gas around the nasal cavity and communicating with the nasal cavity by tubules. Sinus is concealed beside the nasal cavity, wherein the maxillary sinus is located at both sides of the nasal cavity and in the maxillary above orbit; frontal sinus is in the frontal bone; ethmoidal sinus is located at both sides of the upper part of the nasal cavity and is composed of a plurality of small cavities containing gases in the sieve tubes; and sphenoid sinus is in the sphenoid bone behind the nasal cavity.

Clear fluid is secreted from mucosal cells of epidermis within the normal nasal cavity and sinus, then flows into the nasopharynx and throat by virtue of the regular pulse of cilium above mucosal cells from the sinus and through the nasal cavity, and then is swallowed into the esophagus and stomach. Each adult secretes 1L mucus per day, by which the humidity inside the nasal cavity and sinus is guaranteed. At the same time, the mucus can absorb the dust and foreign matter in the air to protect the health of the respiratory tract. The clear mucus will become pus due to the invasion of virus and bacteria, and the stimulation of the foreign matter. The lost of the regular pulse of cilium also will lead to the pus snot or the feeling of postnasal drip. In these cases, the rhinitis or swollen mucosa will be resulted, and the involved tubules will be occluded. Once these tubules are occluded, the nasal mucus will be stagnant in the sinus without emission. These symptoms will evolve into sinusitis, allergic rhinitis or other rhinitis without early treatment.

Sinusitis can cause headaches, nasal congestion, pus snot discharge, temporary olfactory dysfunction, chills, fever, loss of appetite, constipation, aches and discomfort, etc. Symptoms for young children include emesis, diarrhea, and cough, etc. The stimulation of pus snot further causes throat irritation, and sore throat, etc.

As to the allergic rhinitis, once the patient contacts with or inhales allergens, the IgE (immunoglobulin E) in vivo will cause the mast cells to release histamine and thus cause allergic reactions. Allergens are the antigens inducing and reacting with specific IgE antibody. Most allergens are derived from animals, plants, insects, fungus or other specific substances. Allergens can be divided into inhalational allergens and alimentary allergens. The inhalational allergen is the main reason of allergic rhinitis. Symptoms of allergic rhinitis mainly include telangiectasis, increased permeability, increased glandular secretion, and eosinophilic infiltration, etc. Proliferative changes in the mucosa epithelium, mucosal hypertrophy and polypoid lesion will be resulted if above symptoms are recurrent. It has flu-like symptoms, which primarily include nasal itching, nasal congestion, snot, sneezing and watery rhinorrhea (runny nose), etc. These symptoms are intermittent and recurrent with pale edema of nasal mucosa. The worse will evolve into sinusitis, asthma or ear infections.

As to the intranasal therapy by drugs, it is traditional to spay drugs into the nasal cavity by a nasal drop or a nasal spray. Studies show, the effective drugs cannot successfully reach locations of pathological changes by the traditional treatment method due to barriers of tissues, and there are not more than 30% of liquid drugs unevenly reaching locations of pathological changes, thus the effect of therapy by drugs is greatly reduced.

At present, the nasal irrigation device is known to wash the nasal cavity by normal saline or liquid containing drugs. But the short-lived drugs are almost exclusively limited within turbinate, and cannot reach the inside of the frontal sinus and maxillary sinus. Since most of sinus lesions involve any location of the sinus, the nasal irrigation device fails to directly affect the inflammation of the sinus. In fact, even if the liquid drug enters into the sinus, since the ostium of the sinus locates in the bottom, almost all of the liquid drug will discharge through the ostium once the completion of the washing or spraying, wherein drugs have no chance to be stored therein for their long-term effective treatment.

The functional nasal endoscopic surgical procedure is effective for the acute sinusitis and chronic sinusitis. Tissue and bone with pathological changes can be removed precisely to expand the ostium of the sinus and to restore the normal physiological function of the sinus. The nasal endoscopic surgical procedure is minimally invasive compared with the traditional sinusitis surgery. However, nasal endoscopic surgical procedure is expensive and not thorough. The resulted relapse will cause the repeated treatment, which costs a lot and brings heavy mental and economic burdens to patients.

Surgical procedures for treating allergic rhinitis primarily include nerve block surgery, low-temperature plasma surgery, inferior turbinate mucosa surgery, surgery for reducing parasympathetic excitability, and other surgical therapies. But above surgical procedures cause high relapse rate and expensive cost.

The reason for the majority of long-term and repeated nasal diseases lies in: drugs cannot constantly act on locations of pathological changes in a long time, and the intermittent treatment will cause the relapse. If drugs are quantitatively released at locations of pathological changes by simple minimally invasive surgery, the therapy by drugs will be more effective. Although the catheter treatment is convenient, the involved expanded channel is easy to be closed again by the patient's regenerated tissue once the balloon is withdrawn. CN101837148A relates to "a porous biodegradable stent and preparation method thereof', which provides a method for preparing a porous biodegradable stent and for tissue regeneration. However, the pore diameter of the prepared stent is very small, thus the prepared stent can only be used for tissue augmentation rather than providing gas or liquid channel. Thus, the stent cannot support and maintain the expanded state of the ostium of the sinus and thus cannot keep the nasal channel open. Further, in order to achieve such an augmentation purpose, the prepared porous stent has good flexibility and cannot be compressed to be implanted into the nasal cavity through the minimally invasive implantation due to the sterically hindered and other reasons. WO2009/079418 A2 and WO2006/0107957 A2 disclose implant systems for treating sinusitis or allergic rhinitis, wherein the implanted system has a circumferentianally wall formed of a biodegradable polymyer and the wall includes a plurality of openings, with biodegradable fiber bundles containing drugs arranged in the openings. These two implant systems could be of the disadvantage to not be stable enough in their structure and the therapeutic effect is often not satisfying. Therefore, there is a need to find an implant to create an open channel for the ostium of the sinus by minimally invasive surgical intervention, which can sufficiently support the expanded channel in a long time and provide drugs constantly to the sinus under this state in order to radically cure locations of pathological changes and thus to restore the function of the organization and minimize the possibility of being closed again.

### SUMMARY OF THE INVENTION

The invention is based on the problem to provide an implant for treating sinusitis or allergic rhinitis, in order to overcome the problem of the expanded channel being closed again once the balloon is withdrawn and to enhance the structure stability.

This problem is solved by the invention defined in claim 1.
A further solution of the problem is defined in claim 2.
Preferred further embodiments of the invention are defined in the dependent claims.

The present invention provides an implant for treating sinusitis or allergic rhinitis, which can be used after sinus atherectomy or used directly without atherectomy. The implant formed of the biodegradable polymer provided by the invention can provide a sufficient normal force perpendicular to the external surface during a compression, and prevent the supported channel from being closed after a release in the location for the implantation (the location of pathological changes or ostium of the sinus).

Before use, the implant has to be maintained with a relatively smaller diameter to facilitate the routine and delivery of the implant in the nasal cavity. The implant of the invention is provided with openings in the wall, and thus can be compressed to the smaller diameter to be received in a pressure-grip type of delivery catheter or to be bundled to a balloon delivery system. During use, the implant of the invention can be safely and accurately released in the location of pathological changes of the nasal cavity or at the ostium of the sinus by the delivery catheter or the balloon delivery system. The implant of the invention can be a self-expanding implant, or balloon expandable implant, which can be press-griped and constrained in the pressure-grip type of delivery catheter and then delivered to the location of pathological changes by the pressure-grip type of delivery catheter, and thus support the location of pathological changes by self-expanding after the release. The implant of the invention can also be released by a simple conduit in the nasal cavity, which includes one open end located at the ostium of the sinus and the other open end outside of the nose. When the conduit is fixed in the surgery, the compressed implant will be firstly placed at the other open end outside of the nose by hand or by a special device, pushed into the conduit by an elastically bendable rod to the open end located at the ostium of the sinus, and then support the ostium of the sinus by the self-expanding or balloon expanding after the release at the predetermined location. Further, in order to enable the implant to fit the supported tissue, a balloon can be used to help the release (expanding) during the release or after the release. The implant can be pre-tied in the balloon delivery system, which helps to give form to the implant by the expanding balloon for the better fit. After the implant is released by the pressure-grip type of delivery catheter, the balloon can be delivered to support the expanded implant for enhancing the effect of support and fit. If the balloon is used to help the expansion, the used balloon can be a compliant balloon or a semi-compliant balloon, which can be a conventional cylindrical shape or a special shape according to the special application, such as dumbbell-shaped or double tapered balloon.

The present invention provides an implanted system for treating sinusitis or allergic rhinitis, which can ensure the stability and reliability at the position after the implantation, wherein the implantation can be realized, but not limited by the following manner: the implant has a profile including an intermediate portion with an intermediate diameter and two ends with a larger diameter in the axial direction. The implant is "stuck" in the ostium of the sinus after the implantation, thus the frictional force is increased between the external surface of the implant and the expanded location for fixing the implant at the expanded location.

The implant for treating sinusitis or allergic rhinitis of the present invention includes a portion "stuck" in the expanded location, which may be straight- shaped, cylindrical with inconsistent diameters or other special shapes, wherein the preferable shape is dumbbell-shape or double tapered shape having two ends with a slightly larger diameter. The diameter of each end of the implant may be the same or different, i.e., one end with a bigger diameter and the other end with a smaller diameter, but both diameters are not less than the intermediate diameter of the intermediate portion of the implant. Such implant can be "stuck" at the ostium of the maxillary sinus or frontal sinus or the location of pathological changes to prevent the implant from moving due to a motion and other external cause. The intermediate portion of the implant plays a decisive role of support, while two ends of the implant play the role of auxiliary support, better apposition to tissue wall, retention and position definition. The inflamed sinus and sinus tissue or cartilage of the ostium of the sinus are stretched out or deformed by the released implant to form an open drainage channel. If a balloon is used to help the expansion, the drainage channel will be clearer and the implant will fit the expanded tissue better. The balloon including two ends with larger diameters can give form including two ends with larger diameters to the straight-shaped substrate when the implantation or after the implantation. If there is a drug eluting layer on the surface of the implant, compared with the implant with the same length and consistent diameter, the implant having two ends with larger diameters can carry more drugs due to the increased volume and surface area, or the implant with the equivalent drugs will contact with the tissue by the lager contacting area to improve the effect of drug delivery and therapy. Such effect will be strengthened by virtue of the balloon.

The implant for treating sinusitis or allergic rhinitis of the present invention includes, but is not limited to following designs for increasing the friction or retention between the implant and expanded location: the wall of the implant has at least one projection projecting from the external surface, wherein the projection can "penetrate" the expanded tissue to "hang" the implant at the predetermined location for increasing the fixation effect; if the expanded tissue allows, the support force can be appropriately increased to achieve the effect of increasing friction; the external surface of the implant can be modified by adding other substances or removing substances from the external surface to provide the wall with increased roughness or friction coefficient and so on.

Typically, in order to obtain a better fixation effect after the implantation, one or more of above designs can be used.

The present invention provides an implant for treating sinusitis or allergic rhinitis, wherein biodegradable fiber bundles containing drugs are arranged in the first openings of the wall of the implant, wherein some of openings may receive no fiber bundle, and other openings may receive one or more fiber bundles. The biodegradable fiber bundles can be arranged in multiple openings successively in the axial direction or in the circumferential direction (perpendicular to the axial direction) or irregularly to form the fiber bundles inside (axial internal bore) or outside of the implant. The length of the bundle arranged in the axial direction is greater than the absolute axial length of the implant, wherein the preferable length of the bundle is 1.2 times to 10 times of the absolute axial length. The length of the bundle arranged in the circumferential direction along the profile of the wall is greater than the circumference of the arranged implant portion, wherein the preferable length of the bundle is 1.5 times to 5 times of the circumference. The overall length of the irregularly arranged bundle is over 1.2 times greater than the length of the bundle path between one of the starting openings and one of the ending openings, wherein the preferable length of the bundle is 1.5 times to 5 times. The fiber bundle includes at least one continuous fiber. The length of each fiber within the fiber bundle may be the same or different.

Drugs are contained in the fiber bundle or on the surface of the fiber bundle, and drugs for the bundle is the same as or different from the drugs in the drug eluting layer of the wall of the implant. Also, the biodegradable polymer of the fiber bundle can be the same as or different from the biodegradable polymer of the wall of the implant. The biodegradable fiber bundle containing drugs can be obtained by impregnating the biodegradable fiber bundle into the solution of the drugs, or by spaying or bushing the solution of drugs onto the biodegradable fiber bundle. The biodegradable fiber bundle with more stable drugs can be obtained by a spinning process of a biodegradable polymer and a drug solution, wherein drugs are homogeneously mixed with the polymer to obtain a homogeneous fiber bundle containing drugs. Further, the biodegradable fiber bundle with further more stable drugs can be obtained by skin-core fibers containing drugs, wherein drugs and polymers of the skin are different from drugs and polymers of the core, and wherein both the skin and core contain drugs or only the skin or the core contains drugs. Different from impregnating, spraying, brushing and other methods, drugs in such skin-core fibers containing drugs are not only located at the external surface of fibers, but blended with the biodegradable polymer, thus drugs are more stable and have the sustained-release effect.

The fiber bundle will not fall off from the implant during the implantation without the need of tightly wrapping the implant. After the implantation, the biodegradable fiber bundles containing drugs act as many tentacles, thus drugs can reach a large area on both sides of the implant around the expanded location, in particular the cavities within the sinus. The scattered fiber bundles on both sides of the implant provide an expanded treating area of the implant for greatly enhancing the therapeutic effect. Fiber bundle will fall off from the implant in whole or in part after the implantation or after the degradation, and the fiber bundle in the cavities within the sinus will form a fiber group or maintain the shape of the fiber bundle, while other fiber bundles will be transferred to locations of pathological changes, which are not suitable for implanting the implant, by the air flow or the movement of mucus.

### BRIEF DESCRIPTION OF THE DRAWINGS

These and other aspects, features and advantages of the invention will become more readily appreciated when considered in connection with the following detailed description of presently preferred embodiments and best mode, appended claims and accompanying drawings, in which:
Figure 1A is a view of an implanted system for treating sinusitis or allergic rhinitis in accordance with one preferred embodiment of the invention;
Figure 1B shows a position of a monofilament in three-dimensional reference system of the embodiment of Figure 1A ;
Figure 2 is a view of an implanted system for treating sinusitis or allergic rhinitis in accordance with another preferred embodiment of the invention;[00039] Figure 3A is a view of a base body of an implanted system for treating sinusitis or allergic rhinitis in accordance with yet another preferred embodiment of the invention;
Figure 3B is a view of an implanted system for treating sinusitis or allergic rhinitis in accordance with yet another preferred embodiment of the invention;
Figure 3C is a view of an implanted system similar with Figure 3B ;
Figure 4 is a view of an implanted system for treating sinusitis or allergic rhinitis in accordance with yet another preferred embodiment of the invention;
Figure 5A is a view of a base body of an implanted system for treating sinusitis or allergic rhinitis in accordance with yet another preferred embodiment of the invention;
Figure 5B is a view of an implanted system for treating sinusitis or allergic rhinitis in accordance with yet another preferred embodiment of the invention;
Figure 6 is a view of an implanted system for treating sinusitis or allergic rhinitis in accordance with yet another preferred embodiment of the invention;
Figure 7 shows a released effect of the implanted system at the ostium of the maxillary sinus in accordance with yet another preferred embodiment of the invention;
Figure 8 shows biodegradable fiber bundles of the implanted system; and
Figure 9 is an enlarged view of Figure 8 .

### DETAILED DESCRIPTION OF THE PREFERRED EMBODIMENTS

In the text, fiber bundles mean the biodegradable fiber bundles containing drugs arranged in openings of a wall of an implant and include more than one fiber each with a diameter of 100nm-1 00µm; monofilaments for weaving in embodiments 1-3 mean the fibers which are used to weave the wall of the implant and include one or more fiber each with a diameter of 100µm-800µm; support ribs in embodiment 3 mean support members each with a diameter of 400µm-1 mm which are arranged in the preformed openings of a base body and act as a weaving portion for combining the base body and the weaving portion into a unity; fibers for bundling in embodiment 6 are used for bundling the implant to maintain a slender shape (with a smaller diameter) of a curled implant before the implantation and to increase a friction of an external surface of the implant, which include more than one fiber each with a diameter of 1µm-200µm.

### Embodiment 1

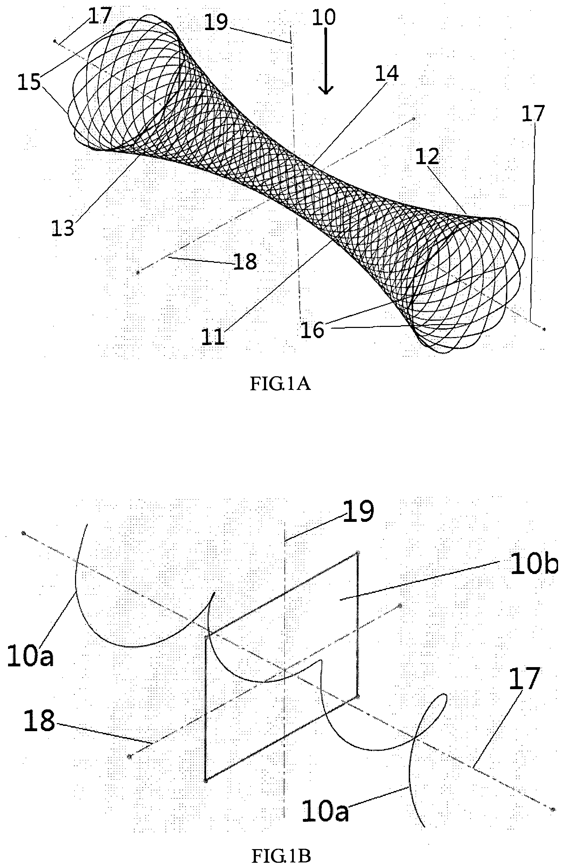

Referring to the Figures 1A-1B, an implant 10 for treating sinusitis or allergic rhinitis in accordance with one preferred embodiment of the invention is woven from monofilaments, which are formed of a biodegradable polymer. The implant 10 has a circumferentially extending wall 11, which is closed in the circumferential direction, namely the wall 11 defines a closed tubular structure in the circumferential direction with two open ends. The wall 11 is woven from monofilaments with a plurality of openings between weaving fibers, thus the wall 11 has a certain degree of flexibility and can be moderately compressed and expanded.

The amount or density of monofilaments for the implant 10, in particular with a same diameter and length, can be controlled by the number of back bending points 15 at two ends of the implant during weaving. The amount/density of monofilaments is larger when the number of back bending points 15 is increased, thus the support force of the implant 10 is bigger when the woven density of the wall 11 is increased. At the same time, the number of monofilament crossing points 16 is changed with the woven density.

The woven wall 11 of the implant 10 has a point, which defines an ex-circle perpendicular to the axial direction along the outer contour of the external surface of the implant, with an initial circumference LO, a compressed circumference L1 after a compression, and a restore circumference L2 after the compression and then released under an unconstrained state. The initial circumference LO is an initial circumference of the woven implant 10 in an initial state without an external force. The compressed circumference L1 is a circumference of the compressed implant in the compressed state after the initial implant 10 compressed by the external force. The restore circumference L2 is a circumference of the released implant 10 in an unconstrained state without the external force from the compressed state. A compression/expansion ratio represented by percentage is defined as a ratio of the restore circumference L2 to the initial circumference LO.

In the present embodiment, both end portions 12 and 13 of the wall 11 have positioning portions extending radially outwardly, i.e., radial lengths of end portions 12, 13 of the wall are greater than a radial length of an intermediate portion 14 between end portions 12, 13. Such implant 10 may have different compression/expansion ratios at different portions, and an overall compression/expansion ratio of the implant 10 can be valued by an average of compression/expansion ratios of points per unit axial length. When the compression/expansion ratio is approaching 100%, the implant 10 has the best resilience, and vice versa, the self-expanding performance is worse when the resilience is decreased.

During weaving, the decisive factors for the woven performance of the implant 10 are the number of back bending points 15 of monofilaments and the turns (i.e., a surrounding helix angle) of monofilaments surrounding from one end to the other end of the wall 11.

As shown in Figure 1A, 17 represents an axis line of the implant 10 in an axial direction and can be regarded as X-axis in a three-dimensional view; 18 represents an axis perpendicular to the axis line 17 through a centre of the implant 10 and can be regarded as Y-axis in a three-dimensional view; 19 represents an axis both perpendicular to the axis line 17 and axis 18 through the centre (an intersection of the X-axis and Y-axis, i.e., the origin) of the implant 10 and can be regarded as Z-axis in a three-dimensional view. The so-called helix angle is a formed acute angle between monofilaments and an YZ plane during weaving. Figure 1B shows a position of a monofilament 1Oa of the woven implant 10 in three-dimensional reference system, wherein the helix angle is the acute angle between the monofilament 1Oa and YZ plane 1Ob. In an implant, the helix angles formed by weaving may be constant or variable.

In practice, the compressed dimension before the implantation and the support state after the implantation can be balanced by adjusting the number of back bending points 15 and the surrounding helix angle. The woven density is smaller when the number of back bending points 15 is less, and an interaction of monofilaments is less when the number of crossing points 16 is decreased, thus the implant can be compressed into smaller radial dimension before the implantation and be easy to be delivered to locations of pathological changes during the implantation, but with the relatively lower support force after the implantation. If turns of monofilaments surrounding from one end to the other end of the contour increased, namely the surrounding helix angle is smaller, the number of crossing points 16 is increased, and thus the implant will require to be compressed into a larger radial dimension before the implantation and can be delivered to wided spaces/cavities of pathological changes during the implantation, but with the higher support force after the implantation. During weaving, the helix angle also affects radial strength, compression force, an axial elongation of the compression and an axial shortening of the release, and these indicators will also affect the processing and performances of the implant.

The implant can be woven by only one or one strand of monofilament, and can also be woven by a plurality of or a plurality of strands of monofilaments. Depending on the weaving design of the mold, along the axial direction, the implant may have a different weaving pattern in a certain section from other sections. The all other section can be woven with same or different weaving pattern.

It should be understood that shapes and positions of positioning portions of this embodiment can be adjusted as desired. The radial length of the implant 10 can be varied along the entire axial direction, e.g., gradually increases from the intermediate portion toward the ends, or suddenly increases at the ends. For example, the implant is dumbbell-shaped or double tapered and includes two ends with larger diameters. The implant can also be designed to have two spherical ends connected by a straight-shaped structure in the middle. The implant 10 is shown as a self-expandable implant, which is compressed into a smaller dimension in a catheter before the implantation, delivered by the catheter, released from the catheter and expanded into an initial dimension and stuck or lock at locations of pathological changes.

In order to obtain a better fixation effect at locations of pathological changes and better enable the implant to fit the supported tissue (locations of pathological changes), a balloon can be used to help the release (expansion) during a release or after the release. The implant can be pre-bundled in a balloon delivery system, which helps to give form to the implant by an expanding balloon for the better fit. After the implant is released by a pressure-grip type of delivery catheter, the balloon can be delivered to support the expanded implant for enhancing the effect of support and fit. If the balloon is used to help the expansion, the used balloon can be a compliant balloon or a semi-compliant balloon, which may have a conventional cylindrical profile or a special shape according to the special application, such as dumbbell-shaped or double tapered balloon.

Since the nasal channel is different from the blood vessel with a closed channel, and there is no blood pressure and other conditions to limit the implant in a relatively stable position, the straight-shaped biodegradable implant has the possibility of displacement after the implantation. Accordingly, the implant including two ends with larger diameters and waist can be "stuck" or lock by conforming around the tissue in the nasal cavity and more adaptable for the nasal cavity. Since the implant has positioning portions extending radially outwardly at the ends of the wall, the positioning portions can be "stuck" in the ostium of the maxillary sinus or frontal sinus or locations of pathological changes, to prevent the implant from moving because of a motion and other external causes. The intermediate portion of the implant plays a decisive role of mechanical support, while two ends of the implant play roles of auxiliary support and position definition. Once the implant is released, the inflamed sinus and sinus tissue or cartilage of the ostium of the sinus is stretched out or deformed to form a smooth drainage channel. Such effect will be enhanced by a balloon-assisted release.

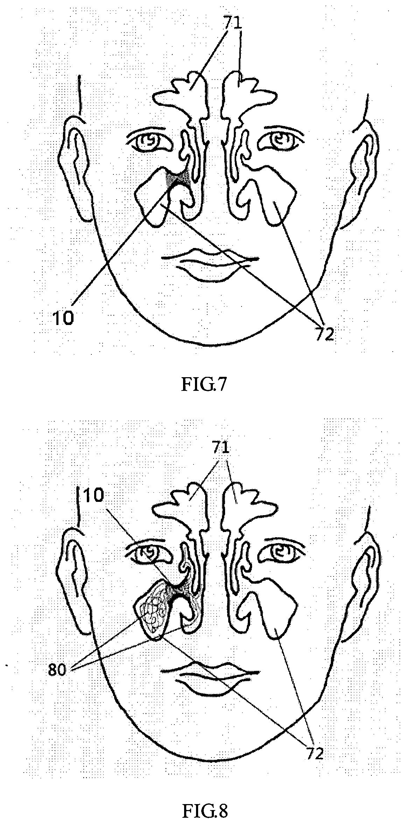

Figure 7 shows a released effect of the implanted system at the ostium of the maxillary sinus in accordance with the present embodiment of the invention. The implant 10 is released at one of the ostium 72 of the maxillary sinus, and the ostium of the sinus is enlarged by shaping (or self-expanding or balloon dilatation). Further, the implant 10 can also be released at the ostium of the frontal sinus 71. In addition to a good fixation of the implant 10, such design can also increase the contact area between the external surface of the implant 10 and the tissue. If there is a drug eluting layer containing drugs on the external surface of the implant 10, drugs of the drug eluting layer of this design will have much better contact with the tissues of pathological changes due to a lager contacting area to improve the effect of drug delivery and therapy.

In order to obtain a better effect of drug release and treatment, biodegradable fiber bundles 80 containing drugs are arranged in openings between the monofilaments of the wall 11 of the implant 10 in this embodiment. The arranging mode of biodegradable fiber bundles 80 can be varied as desired and thus not shown in the drawings. For example, the fiber bundles are arranged through the openings between monofilaments in the axial direction, or the fiber bundles are arranged through the openings between monofilaments in the circumferential direction, or the fiber bundles are arranged through the openings between monofilaments irregularly and thus forming the "tentacles" extending from the implant. The fiber bundle includes at least one continuous fiber. Every fiber within the fiber bundle can have the same or different length. Drugs are contained inside or on the surface of the bundle. The fiber bundle may wrap the implant with a certain rigid thus it will not fall off from the implant during the implantation without the need of tightly wrapping the implant.

As shown in Figure 8, after the implant 10 is located at the ostium of the maxillary sinus 72, the fiber bundles 80 carried on the implant are scattered on both sides of the implant 10 (namely in both sides of the ostium of the sinus). The biodegradable fiber bundles 80 containing drugs act as many tentacles, thus drugs can reach the cavities and channels (which are not directly contacted with the body of the implant) within the maxillary sinus 72. The scattered fiber bundles 80 on both sides of the implant expand the treating area of the implant for greatly enhancing the therapeutic effect.

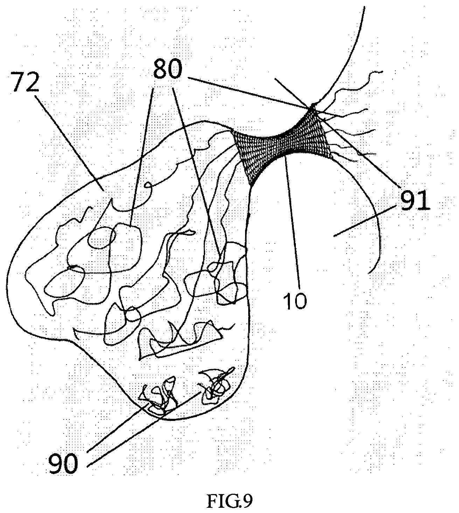

Figure 9 is a partial enlarged view of Figure 8 , wherein the ostium tissue of the maxillary sinus 72 is shown by 91. Preferably, fiber bundles 80 will fall off from the implant 10 in whole or in part after the implantation or after the degradation of the implant 10, and the fiber bundles in the cavities within the sinus will form a fiber group 90 or maintain the shape of fiber bundle, while other fiber bundles will be transferred to locations of pathological changes, which are not suitable for implanting the implant, by the air flow or the movement of mucus.

When in a compressed state, the implant 10 is shown as cylindrical, and the radial length (diameter) of each point is substantially the same, between 0.5-10 mm, preferably between 2mm and 5mm. Since the radial length is reduced in the compressed state, the corresponding axial length of the implant will increase, and the extend length is dependent on the graphics structure design and constrained diameter, i.e., the axial length can be varied with the specific applications. When in a released unconstrained state, the radial length of the intermediate portion 14 of the implant 10 is 2 mm-30 mm, the radial lengths of the end portions 12, 13 of the implant 10 are 2mm-30mm larger than the radial length of the intermediate portion 14, and the exemplary axial length of the implant 10 is 2mm-60mm.

### Embodiment 2

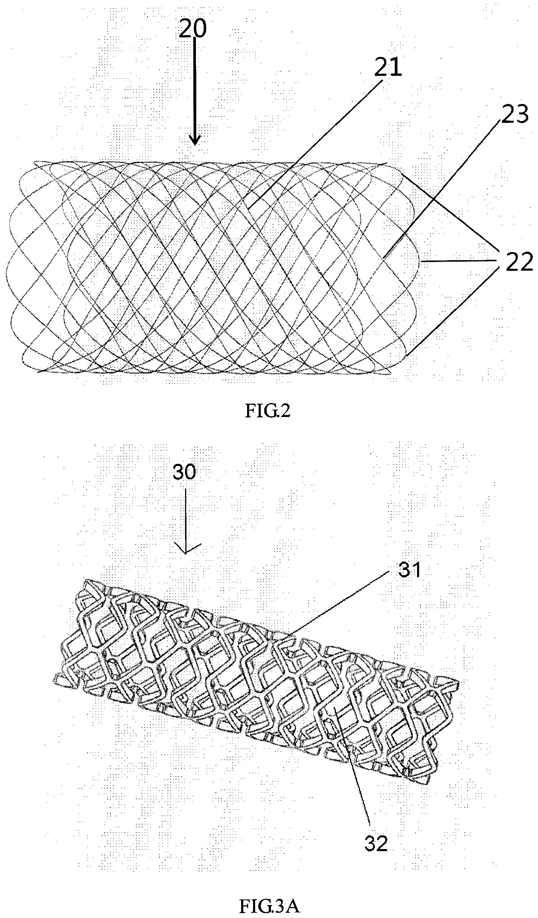

As shown in Figure 2, an implant 20 for treating sinusitis or allergic rhinitis in accordance with another preferred embodiment of the invention is woven from monofilaments, which are formed of the biodegradable polymer. The implant 20 has a circumferentially extending wall 21, which is closed in the circumferential direction, namely the wall 21 defines a closed tubular structure in the circumferential direction with two open ends. The wall 21 is woven from monofilaments with a plurality of openings therebetween, thus the wall 21 has a certain degree of flexibility and can be moderately compressed and expanded. As discussed in above embodiment, the properties of the implant, such as compressibility before the implantation, delivery ability during the implantation, the therapeutic effect after the implantation, etc., can be balanced by adjusting number of the back bending points 22 at ends and the surrounding helix angle of monofilaments during weaving. If the number of the back bending points 22 is increased, the number of crossing points 23 and the interaction of monofilaments are more, and thus the implant will require to be compressed into a larger radial dimension before the implantation and have challenge to be delivered to smaller space of pathological changes during the implantation, but with the higher support force after the implantation. If the surrounding helix angle is increased, and the woven density is smaller, then the number of crossing points 23 and the interaction of monofilaments are decreased, the implant can be compressed into a smaller radial dimension before the implantation and be easy to be delivered to smaller locations of pathological changes during the implantation, but with the relatively lower support force after the implantation.

The implant can be woven by only one or one strand of monofilament, and can also be woven by a plurality of or a plurality of strands of monofilaments depending on the weave design of the mold. In order to have different performance and effects at the different section, the implant may have a different weaving pattern in a certain section from another section along the axial direction, which can be woven with same or different weaving pattern from other sections. The corresponding advantages include: the implant can be given the form with different diameters at different sections by a specific balloon in order to better fit the irregular tissue surface. For example, both ends of the implant are woven into the patterns easier to be expanded, thus although the woven implant is cylindrical with a constant diameter at the initial, the implant can be expanded to the form including two ends with larger diameters by the balloon-assisted release and smaller, high density waist will be locked at the narrow location of the cavity.

In the present embodiment, biodegradable fiber bundles containing drugs can also be arranged in openings between monofilaments of the wall 21 of the implant 20, which are arranged through the openings in the axial direction, or in the circumferential direction, or irregularly and thus forming the "tentacles" extending from the implant. Other features of the implant 20 which are not described in detail are the same as the embodiment 1 and will not be repeated here.

The implant 20 is shown as a self-expandable implanted system. When in the unexpanded (compressed) state, the implant 20 is shown as cylindrical with a diameter of 0.5mm-1 Omm. When in the expanded (released unconstrained) state, an external diameter of the implant 20 is 2mm-30mm, and an axial length is 2mm-60mm.

### Embodiment 3

As shown in Figures 3A-3B, an implant for treating sinusitis or allergic rhinitis in accordance with yet another preferred embodiment of the invention comprises a cylindrical base body 30. A wall 31 of the base body 30 has interspaces 32 formed by laser cutting. The implanted system also comprises extended end portions, which are woven by monofilaments arranged in interspaces 32 axially outwardly from the ends of the base body 30.

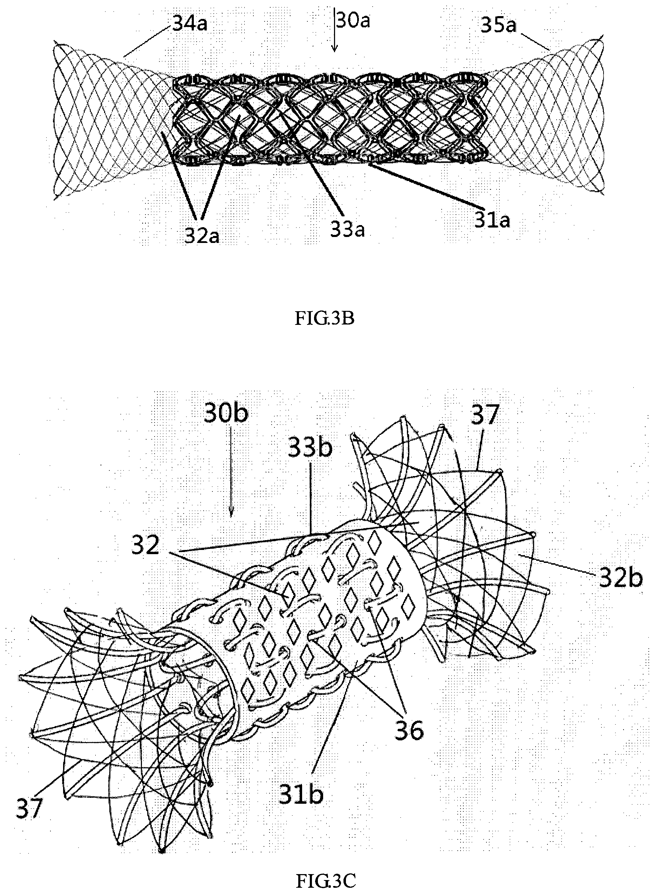

Figure 3A shows the base body 30 with a constant diameter formed of a laser cut hollow tube, wherein base body 30 with the constant diameter has a circumferentially extending wall 31, which is closed in the circumferential direction, namely the wall 31 defines a closed tubular structure in the circumferential direction with two open ends, i.e., the base body 30 with the constant diameter is formed as a cylindrical structure. The wall 31 is a plate-like structure of a biodegradable polymer, and then interspaces 32 with various shapes and spaces are formed in the wall 31 by laser cutting. Monofilaments of the biodegradable polymer is arranged in interspaces 32 and woven from both ends of the base body 30 with the constant diameter to form the positioning portions with larger diameters, as shown in Figure 3B .

Figure 3B shows a segmented implant 30a of the present embodiment, the intermediate portion of the implant is the base body 30 formed by laser cutting (see Fig 3A ), and both ends of the implant are the extended end portions 34a, 35a woven of monofilaments. There are openings 32a between the woven monofilaments of the extended end portions. Due to the openings 32 of the base body 30 and the openings 32a of the extended end portions 34a, 35a, the wall 31a of the segmented implant 30a has a certain degree of flexibility and can be moderately compressed and expanded. The extended end portions 34a, 35a form positioning portions extending radially outwardly, i.e., radial lengths of the extended end portions 34a, 35a are greater than the radial length of the intermediate base body 30.

The woven monofilaments may be only arranged in the openings 32 of both ends of the base body 30 as a linker, and also may be arranged throughout the base body 30, i.e. the inside (inner wall) or outside (outer wall) of the base body comprises woven monofilaments 33a. If there are woven monofilaments 33a inside the base body, the base body and the woven portions form a better unity.

In the present embodiment of Figure 3B, biodegradable fiber bundles containing drugs are also arranged in openings 32, 32a of the wall 31a of the implant 30a, which are arranged through the openings in the axial direction, or in the circumferential direction, or irregularly and thus forming the "tentacles" extending from the implant. Other features of the implant 30a which are not described in detail are the same as the embodiment 1 and will not be repeated here.

As shown in Figure 3C, besides the cutting interspaces 32, there are preformed apertures 36 on the base body 30. Support ribs 33b of a biodegradable polymer are arranged in the preformed apertures 36 and have a much bigger diameter than the diameter of woven monofilaments 37. Support ribs 33b are arranged in the preformed apertures 36 before weaving, and then the support ribs 33b outwardly from the ends of the base body 30 are heat set into trumpet-shaped. Monofilaments 37 are woven from the ends of the base body 30 and attached to the support ribs 33b. There are interspaces 32b between monofilaments 37 and support ribs 33b. This design allows for the overall homogeneity and better firmness of the implant 30b.

In the present embodiment of Figure 3C, biodegradable fiber bundles containing drugs are also arranged in openings 32, 32b of the wall 31b of the implant 30b, which are arranged through the openings in the axial direction, or in the circumferential direction, or irregularly and thus forming the "tentacles" extending from the implant. Other features of the implant 30b which are not described in detail are the same as the embodiment 1 and will not be repeated here.

The implants 30a, 30b are shown as self-expandable implanted systems. When in the unexpanded (compressed) state, the implant 30 is shown as cylindrical with a diameter of 0.5mm-10mm. When in the expanded (released unconstrained) state, an external diameter of the implant 30 is 2mm-30mm, and an axial length is 2mm-60mm.

### Embodiment 4

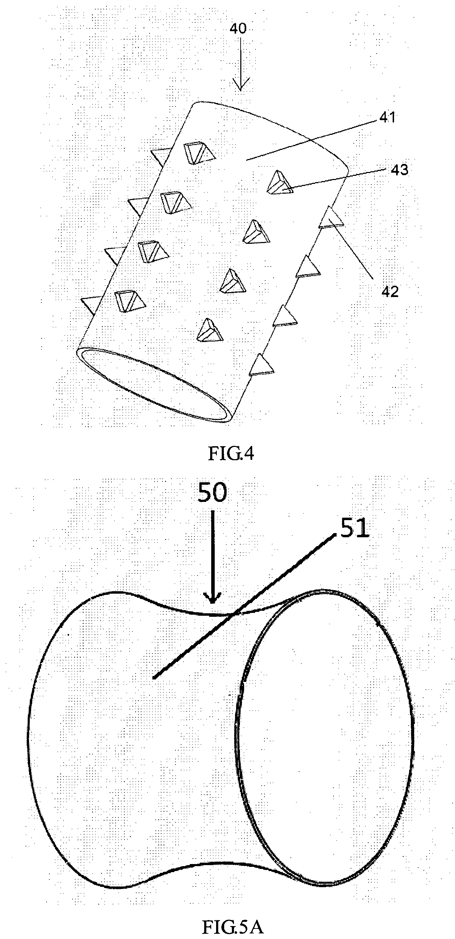

As shown in Figure 4, an implant for treating sinusitis or allergic rhinitis in accordance with yet another preferred embodiment of the invention is a tubular implant 40. The implant 40 has a circumferentially extending wall 41, which is closed in the circumferential direction, namely the wall 41 defines a closed tubular structure with two open ends, i.e., the implant 40 is formed as a cylindrical structure. The wall 41 is a plate-like structure of a biodegradable polymer, and then projections 42 extending radially outwardly from the external surface of the wall 41 may be formed by laser cutting, and wherein openings 43 are correspondingly formed. In the present embodiment, two sides of a triangle in the wall 41 are laser cut, and then the triangle is artificially folded along the uncut side of the triangle. The wall 41 can be compressed and expanded due to the toughness of fibers and interspaces 43 under an external force. It should be understood that shapes and positions of projections 42 and the corresponding interspaces 43 can be adjusted as desired. The implant 40 is shown as a self-expandable implanted system. When in the unexpanded (compressed) state, the implant 40 is shown as cylindrical with a diameter of 0.5mm-1 0mm. When in the expanded (released unconstrained) state, a radial length of the implant 40 is 2mm-30mm, and a axial length is 2mm-60mm. When the implant is press-griped, projections are bent within the surface of the implant under the external force. When the implant is released, projections are pushed outwardly from the external surface of the implant by a balloon and form "thorns", at least one of which penetrates the nasal surface in order to fix the implant in position. "Thorns" can be formed only in the intermediate portion of the implant, and when such implant is expanded by a spherical or ellipsoidal balloon, a diameter of the intermediate portion is larger than the diameters of ends of the implant. In this case, "thorns" are more easily stretched out.

In the present embodiment, biodegradable fiber bundles containing drugs are also arranged in openings 43 of the wall 41 of the implant 40, which are arranged through the openings in the axial direction, or in the circumferential direction, or irregularly and thus forming the "tentacles" extending from the implant. Other features of the implant 40 which are not described in detail are the same as the embodiment 1 and will not be repeated here.

### Embodiment 5

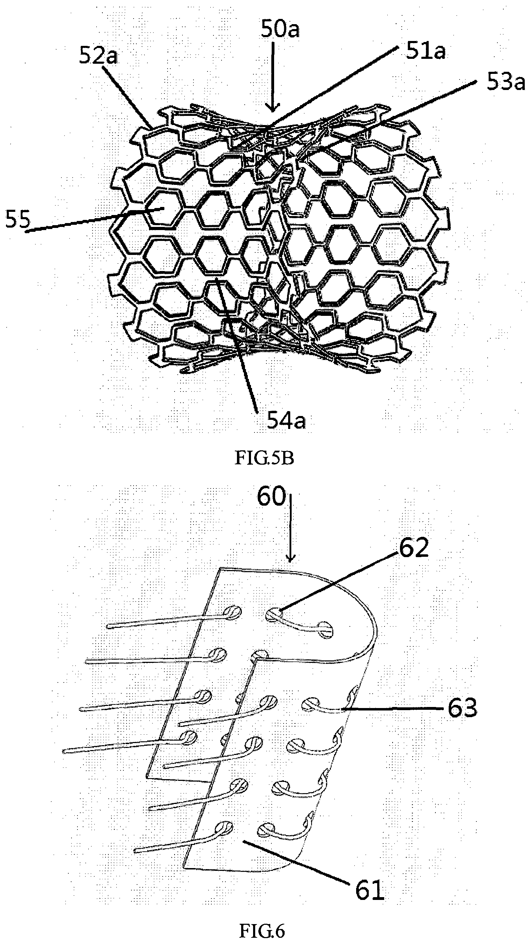

As shown in Figures 5A-5B, an implant for treating sinusitis or allergic rhinitis in accordance with yet another preferred embodiment of the invention is a tubular implant 50a. As shown in Figure 5B, the implant 50a has a circumferentially extending wall 51a, which is closed in the circumferential direction, namely the wall 51a defines a closed tubular structure with two open ends, i.e., the implant 50a is formed as a cylindrical structure. The wall 51a is firstly formed as a tubular base body 50 of a biodegradable polymer, and then the openings 55 with various shapes and spaces are formed in the initial wall 51 by laser cutting or the like, thus the wall 51a can be compressed and expanded under an external force. The wall 51a has positioning portions extending radially outwardly at both end portions 52a, 53a of the wall, namely radial lengths of end portions 52a, 53a of the wall is bigger than the radial length of the intermediate portion 54a. In present embodiment, the wall 51a has trumpet-shaped openings at both ends. It should be understood that shapes and positions of the positioning portions can be adjusted as desired. The implant 50a is shown as a self-expandable implanted system. When in the unexpanded (compressed) state, the implant 50a is shown as cylindrical with a diameter of 0.5mm-1 0mm. When in the expanded (released unconstrained) state, a radial length of the intermediate portion 54a of the implant 50a is 2mm-30mm, radial lengths of end portions 52a, 53a of the implant 50a are 2mm-30mm bigger than the radial length of the intermediate portion, and an axial length of the implant 50a is 2mm-60mm. The implant with both trumpet-shaped ends can be obtained by a trumpet-shaped balloon, and also can be obtained by cutting a tubular material with a constant diameter and expanding the ends by the trumpet-shaped balloon, wherein 1/5-1/3 of the length is set as with slightly bigger diameters, and the intermediate portion is set as with a smaller diameter. The biodegradable material can also be used to form biodegradable nonwoven or woven flat screen, and then which can be given the form of straight or with trumpet-shaped openings by welding or sewing. It should be appreciated that the implant can be obtained by one or more method from filming, casting, weaving, winding, laser cutting, or other processing manners.

In the present embodiment, biodegradable fiber bundles containing drugs are also arranged in openings 55 of the wall 51a of the implant 50a, which are arranged through the openings in the axial direction, or in the circumferential direction, or irregularly and thus forming the "tentacles" extending from the implant. Other features of the implant 50a which are not described in detail are the same as the embodiment 1 and will not be repeated here.

### Embodiment 6

As shown in Figure 6 , an implanted system for treating sinusitis or allergic rhinitis in accordance with yet another preferred embodiment of the invention is an implant 60. The implant 60 has a circumferentially extending wall 61, which is non-closed in the circumferential direction and is shown as C-shaped, wherein the arc portion of the wall 61 takes up 60-360 degrees in the overall circumference. The wall 61 is firstly formed as a plate-like structure of a biodegradable polymer, and then uniformly distributed apertures 62 (also can be not uniformly distributed) are formed in the wall by laser cutting with biodegradable fibers 63 arranged in openings. In the present embodiment, the wall 61 may be curled by a sheet substrate, wherein the openings 62 can be precut in the sheet substrate, and also can be obtained by cutting the curled sheet substrate. The wall 61 can be compressed and expanded under an external force due to the toughness of the polymer sheet and second openings 62. On the other hand, compression performance and expansion capability are more flexible due to the tension of the non-closed wall 61 in the circumferential direction. When the implant is press-griped, the wall 61 can be curled and bundled by the biodegradable fiber 63 to limit the diameter of the implant in a small range. It should be understood that shapes and positions of the openings 62 and fibers 63 can be adjusted as desired. The implant 60 is shown as a self- expandable implanted system, which is compressed in a catheter before the implantation, delivered through the catheter by a doctor, released from the catheter and expended into initial dimension to support locations of pathological changes. The implant can be bundled on a balloon and expanded at locations of pathological changes by the balloon. When in the unexpanded (compressed) state, the implant 60 is shown as cylindrical with a diameter of 0.5mm-10mm. When in the expanded (released unconstrained) state, a radial length of the implant 60 is 2mm-30mm, and an axial length is 2mm-60mm. In the processing, the implant can be reduced into a smaller dimension by tightening the polymer fibers and curling the sheet substrate, and then be bundled by the arranged fibers and fixed to the delivery system, and finally be released after the implantation. The advantage of such sheet support includes the strong supporting force. The implant fits the tissue and firmly retains at location of pathological changes without the ends with large diameters. Of course, arranged fibers can increase the friction between the support and location of pathological changes and play a positive role in the fixation.

In the present embodiment, biodegradable fiber bundles containing drugs are also arranged in openings 62 of the wall 61 of the implant 60, which are arranged through the openings in the axial direction, or in the circumferential direction, or irregularly and thus forming the "tentacles" extending from the implant. Other features of the implant 60 which are not described in detail are the same as the embodiment 1 and will not be repeated here. Certainly the biodegradable fibers 63 containing drugs can enhance the therapeutic effect.

Examples of the biodegradable material of the substrate of the implant of the implanted system for treating sinusitis of the invention include, but are not limited to the following polymers: polylactic acid (PLA), L-polylactic acid (PLLAor LPLA), polyglycolic acid / polylactic acid (PGLA), polycaprolactone (PCL), polyhydroxylbutyrate valerate (PHBV), polyacetylglutamicacid (PAGA), polyorthoesters (POE) and polyethylene oxide / polybutylene terephthalate (PEO/PBTP), poly-p-dioxanone (PPDO), and copolymers or blends of the above materials.

The implant for treating sinusitis of the invention includes a external surface with eluting layer, which is biodegradable polymer selected from low molecular weight polymer or mixture of above substrate material of the implant. Preferred material is the low molecular weight polymer or homologues of the low molecular weight polymer of substrate material of the implant, in order to guarantee the good compatibility between the eluting layer and substrate of the implant.

The implant for treating sinusitis of the invention can be an uncoated implant or an implant with drug eluting layer including drugs as desired, wherein the drug eluting layer can be formed by ultrasonic spray coating, manual brushing, immersion or mechanical dispensing and so on.

Drugs in the drug eluting layer can be specific diagnostic agents. Examples of such diagnostic agents include radio-opaque materials such as iodine or iodine-derivatives, for example, iohexal and iopamidol. Other diagnostic agents such as, for example, radioisotopes, are detectable by tracing radioactive emissions. Examples of agents detectable by MRI are generally paramagnetic agents including, but are not limited to, gadolinium chelated compounds. An example of an agent detectable by ultrasound includes, but is not limited to, perflexane. An example of a fluorescence agent includes, but is not limited to, indocyanine green. Examples of agents used in diagnostic PET include, but are not limited to, fluorodeoxyglucose, sodium fluoride, methionine, choline, deoxyglucose, butanol, raclopride, spiperone, bromospiperone, carfentanil, and flumazenil.

Drugs in the drug eluting layer can be selected from, but are not limited to, following therapeutic agent: anti-inflammatory agents, anti-allergens, anti-cholinergic agents, antihistamines, anti-infectives, anti-platelet agents, anti-coagulants, antithrombotic agents, anti-scarring agents, anti-proliferative agents, chemotherapeutic agents, anti-neoplastic agents, decongestants, healing promoting agents and vitamins (for example, retinoic acid, vitamin A, depaxapanthenol, vitamin B and their derivatives), hypersomolar agents, immunomodulators, immunosuppressive agents, and combinations and mixtures thereof.

Anti-infective agents generally include antibacterial agents, antifungal agents, antiparasitic agents, antiviral agents, and antiseptics. Anti-inflammatory agents generally include steroidal and nonsteroidal anti-inflammatory agents.

Examples of antiallergic agents that may be suitable for use with the implant of the invention include, but are not limited to, pemirolast potassium (ALAMAST^{®}, Santen, Inc.), and any prodrugs, metabolites, analogs, homologues, congeners, derivatives, salts and combinations thereof. Examples of antiproliferative agents include, but are not limited to, actinomycin D, actinomycin IV, actinomycin 11, actinomycin X1, actinomycin C1, and dactinomycin (COSMEGEN^{®}, Merck & Co., Inc.). Examples of antiplatelet, anticoagulant, antifibrin, and antithrombin agents include, but are not limited to, sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonist antibodies, recombinant hirudin, and thrombin inhibitors (ANGIOMAX^{®}, Biogen, Inc.), and any prodrugs, metabolites, analogs, homologues, congeners, derivatives, salts and combinations thereof.

Examples of cytostatic or antiproliferative agents that may be suitable for use with the implant of the invention include, but are not limited to, angiopeptin, angiotensin converting enzyme inhibitors such as captopril (CAPOTEN^{®} and CAPOZIDE^{®}, Bristol-Myers Squibb Co.), cilazapril or lisinopril (PRINIVIL^{®} and PRINZIDE^{®}, Merck & Co., Inc.); calcium channel blockers such as nifedipine; colchicines; fibroblast growth factor (FGF) antagonists, fish oil (omega 3-fatty acid); histamine antagonists; lovastatin (MEVACOR^{®}, Merck & Co., Inc.); monoclonal antibodies including, but not limited to, antibodies specific for Platelet-Derived Growth Factor (PDGF) receptors; nitroprusside; phosphodiesterase inhibitors; prostaglandin inhibitors; suramin; serotonin blockers; steroids; thioprotease inhibitors; PDGF antagonists including, but not limited to, triazolopyrimidine; and nitric oxide, and any prodrugs, metabolites, analogs, homologues, congeners, derivatives, salts and combinations thereof.

Examples of antibacterial agents that may be suitable for use with the implant of the invention include, but are not limited to, aminoglycosides, amphenicols, ansamycins, β-lactams such as penicillins, lincosamides, macrolides, nitrofurans, quinolones, sulfonamides, sulfones, tetracyclines, vancomycin, and any of their derivatives, or combinations thereof. Examples of penecillins that may be suitable for use with the implant of the invention include, but are not limited to, amdinocillin, amdinocillin pivoxil, amoxicillin, ampicillin, apalcillin, aspoxicillin, azidocillin, azlocillin, bacampicillin, benzyl penicillinic acid, benzylpenicillin sodium, carbenicillin, carindacillin, clometocillin, cloxacillin, cyclacillin, dicloxacillin, epicillin, fenbenicillin, floxacillin, hetacillin, lenampicillin, metampicillin, methicillin sodium, mezlocillin, nafcillin sodium, oxacillin, penamecillin, penethamate hydriodide, penicillin G benethamine, penicillin G benzathine, penicillin G benzhydrylamine, penicillin G calcium, penicillin G hydrabamine, penicillin G potassium, penicillin G procaine, penicillin N, penicillin 0, penicillin V, penicillin V benzathine, penicillin V hydrabamine, penimepicycline, phenethicillin potassium, piperacillin, pivampicillin, propicillin, quinacillin, sulbenicillin, sultamicillin, talampicillin, temocillin, and ticarcillin.

Examples of antifungal agents suitable for use with the implant of the invention include, but are not limited to, allylamines, imidazoles, polyenes, thiocarbamates, triazoles, and any of their derivatives. Antiparasitic agents that may be employed include, but are not limited to, atovaquone, clindamycin, dapsone, iodoquinol, metronidazole, pentamidine, primaquine, pyrimethamine, sulfadiazine, trimethoprim/sulfamethoxazole, trimetrexate, and combinations thereof.

Examples of antiviral agents suitable for use with the implant of the invention include, but are not limited to, acyclovir, famciclovir, valacyclovir, edoxudine, ganciclovir, foscamet, cidovir (vistide), vitrasert, formivirsen, HPMPA (9-(3-hydroxy-2-phosphonomethoxypropyl)-adenine), PMEA (9-(2-phosphonomethoxyethyl)-adenine), HPMPG (9-(3-Hydroxy-2-phosphonomethoxypropyl)-guanine), PMEG (9-[2-(phosphonomethoxy)ethyl]-guanine), HPMPC (1-(2-phosphonomethoxy-3-hydroxypropyl)-cytosine), ribavirin, EICAR (5-ethynyl-1-beta-D-ribofuranosylimidazole-4-carboxamine), pyrazofurin (3-[beta-D-ribofuranosyl]-4-hydroxypyrazole-5-carboxamine), 3-Deazaguanine, GR-92938X (1-beta-D-ribofuranosylpyrazole-3,4-dicarboxamide), LY253963 (1,3,4-thiadiazole-2-yl-cyanamide), RD3-0028 (1,4-dihydro-2,3-benzodithiin), CL387626 (4,4'-bis[4,6-d][3-ami nophenyl-N, N-bis(2-carbamoylethyl)-sulfonil imino]-1 ,3,5-tri azin-2-ylami no-biphenyl--2-,2'-disulfonic acid disodium salt), BABIM (Bis[5-amidino-2-benzimidazoly-1]-methane), NIH351, and combinations thereof.

Examples of antiseptic agents suitable for use with the implant of the invention include, but are not limited to, alcohol, chlorhexidine, iodine, triclosan, hexachlorophene, and silver-based agents, for example, silver chloride, silver oxide, and silver nanoparticles.

Anti-inflammatory agents may include steroidal and nonsteroidal anti-inflammatory agents. Examples of suitable steroidal anti-inflammatory agents include, but are not limited to, 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, any of their derivatives, and combinations thereof.

Examples of suitable nonsteroidal anti-inflammatory agents include, but are not limited to, COX inhibitors. These COX inhibitors may include COX-1 or COX nonspecific inhibitors such as, for example, salicylic acid derivatives, aspirin, sodium salicylate, choline magnesium trisalicylate, salsalate, diflunisal, sulfasalazine and olsalazine; para-aminophenol derivatives such as acetaminophen; indole and indene acetic acids such as indomethacin and sulindac; heteroaryl acetic acids such as tolmetin, dicofenac and ketorolac; arylpropionic acids such as ibuprofen, naproxen, flurbiprofen, ketoprofen, fenoprofen and oxaprozin; anthranilic acids (fenamates) such as mefenamic acid and meloxicam; enolic acids such as the oxicams (piroxicam, meloxicam) and alkanones such as nabumetone. The COX inhibitors may also include selective COX-2 inhibitors such as, for example, diaryl-substituted furanones such as rofecoxib; diaryl-substituted pyrazoles such as celecoxib; indole acetic acids such as etodolac and sulfonanilides such as nimesulide).

Examples of chemotherapeutic/antineoplastic agents that may be used in the implant of the invention include, but are not limited to antitumor agents (e.g., cancer chemotherapeutic agents, biological response modifiers, vascularization inhibitors, hormone receptor blockers, cryo-therapeutic agents or other agents that destroy or inhibit neoplasia or tumorigenesis) such as alkylating agents or other agents which directly kill cancer cells by attacking their DNA (e.g., cyclophosphamide, isophosphamide), nitrosoureas or other agents which kill cancer cells by inhibiting changes necessary for cellular DNA repair (e.g., carmustine (BCNU) and lomustine (CCNU)), antimetabolites or other agents that block cancer cell growth by interfering with certain cell functions, usually DNA synthesis (e.g., 6-mercaptopurine and 5-fluorouracil (5FU), antitumor antibiotics and other compounds that act by binding or intercalating DNA and preventing RNA synthesis (e.g., doxorubicin, daunorubicin, epirubicin, idarubicin, mitomycin-C and bleomycin), plant (vinca) alkaloids and other anti-tumor agents derived from plants (e.g., vincristine and vinblastine), steroid hormones, hormone inhibitors, hormone receptor antagonists and other agents which affect the growth of hormone-responsive cancers (e.g., tamoxifen, herceptin, aromatase ingibitors such as aminoglutethamide and formestane, triazole inhibitors such as letrozole and anastrazole, steroidal inhibitors such as exemestane), antiangiogenic proteins, small molecules, gene therapies and/or other agents that inhibit angiogenesis or vascularization of tumors (e.g., meth-1, meth-2, thalidomide), bevacizumab (Avastin), squalamine, endostatin, angiostatin, Angiozyme, AE-941 (Neovastat), CC-5013 (Revimid), medi-522 (Vitaxin), 2-methoxyestradiol (2ME2, Panzern), carboxyamidotriazole (CAI), combretastatin A4 prodrug (CA4P), SU6668, SU11248, BMS-275291, COL-3, EMD 121974, IMC-1C11, IM862, TNP-470, celecoxib (Celebrex), rofecoxib (Vioxx), interferon alpha, interleukin-12 (IL-12) or any of the compounds identified in Science Val. 289, Pages 1197-1201 (Aug. 17,2000 ), which is expressly incorporated herein by reference, biological response modifiers (e.g., interferon, bacillus calmette-guerin (BCG), monoclonal antibodies, interleukin 2, granulocyte colony stimulating factor (GCSF), etc.), PGDF receptor antagonists, herceptin, asparaginase, busulphan, carboplatin, cisplatin, carmustine, chlorambucil, cytarabine, dacarbazine, etoposide, flucarbazine, flurouracil, gemcitabine, hydroxyurea, ifosphamide, irinotecan, lomustine, melphalan, mercaptopurine, methotrexate, thioguanine, thiotepa, tomudex, topotecan, treosulfan, vinblastine, vincristine, mitoazitrone, oxaliplatin, procarbazine, streptocin, taxol or paclitaxel, taxotere, azathioprine, docetaxel analogs/congeners, derivatives of such compounds, and combinations thereof.

When the implant of the invention is implanted in the nasal cavity, the drug in the drug eluting layer can be released constantly to treat locations of pathological changes, with the slow release property. The amount of drug released from the implant will depend on the desired dosage. Each drug should be released at a rate that provides a patient with a healthy, safe, and effective dosage and should be administered at a dosage that is also healthy, safe, and effective. In some variations, for example when the implant is used to treat one or more conditions of the sinuses, the implant may be configured to deliver mometasone furoate at a daily dosage of about 60 µg or less per day.

Drugs may be released at a constant rate from the implant, but need not be. Indeed, the implant may be configured with any suitable release rate. Multiple drug eluting layers may be used, and each layer may be configured to have a different and specific release rate. Similarly, polymer filaments comprising drug particles may be used to provide multi-layers and each layer with a different release rate. Additionally, as described below, drug depots may be used to achieve a varied release rate. These variations, and combinations thereof, may allow the implant to provide a variable drug release rate, or provide either instantaneous or delayed bursts in addition to the implant's baseline release. One or more release rate modifiers may also be used. The release rate modifier may be any suitable biocompatible materials that serve to alter the rate at which a drug is released from the implant. In some variations, the release rate modifier may include a hydrophilic agent. In some variations, the release rate modifier is a polyethylene glycol, e.g., a polyethylene glycol with a molecular weight of between about 5000 to about 7000, such as PEG 6000.

In some variations, the implant may be configured to deliver multiple drugs, which drugs may or may not be encapsulated (e.g., in a microreservoir or other material). In some variations, multiple types of drug particles are contained within a single drug eluting layer. In other variations, the drug eluting layer is discontinuous, having different sections containing different drugs. In these variations, the different sections may have different compositions, and thus may also provide different release rates. In still other variations, multiple drug eluting layers may be used, where each layer contains a different drug or combination of drugs.

Drug depots, as described above, may also hold different drugs therein or may collectively release different drugs than those released by the drug eluting layer. In still other variations, the drug depots may release a different drug or combination of drugs than those drugs released by the drug eluting layer or layers. Any combination of these variations may also be used to achieve the desired drug delivery profiles.

As required, the supporting time of the implant implanted in the nasal cavity can be controlled by adjusting the degradation property of the substrate material of the implant, which is generally 1-12 months. When the implant is implanted in the nasal cavity, drugs in the drug eluting layer will be released constantly to treat locations of pathological changes directly and continuously, with the purpose of inhibiting the growth of pathological changes and gradual elimination, in order to treat thoroughly sinusitis and other nasal cavity illnesses. The release time and dosage of drugs can be adjusted as desired, wherein the release time can be generally 1 week to 6 months.

## Claims

1. An implant (30) for treating sinusitis or allergic rhinitis, wherein the implant (30) has a circumferentially extending wall (31) formed of a biodegradable polymer, and the wall (31) includes a plurality of openings (32), with biodegradable fiber bundles containing drugs arranged in the openings (32)
**characterized in**
**that** biodegradable fiber bundles are extending from the implant (30) and are scattered on both sides of the implant (30),
wherein the wall (31) is defined by a tubular structure, which is closed in the circumferential direction and which is open at both ends, wherein the wall has uniformly distributed openings (32), with support ribs (33) arranged in the openings (32).

2. An implant (60) for treating sinusitis or allergic rhinitis, wherein the implant (60) has a circumferentially extending wall (61) formed of a biodegradable polymer, and the wall (61) includes a plurality of openings (62), with biodegradable fiber bundles containing drugs arranged in the openings (62)
**characterized in**
**that** biodegradable fiber bundles are extending from the implant (60) and are scattered on both sides of the implant (60),
wherein the wall (61) is non-closed in the circumferential direction and has uniformly distributed openings (62), with biodegradable fibers for bundling arranged in the openings (62).

3. The implant of claim 1, wherein the wall (31) is formed by monofilament weaving, tubular substrate cutting or sheet substrate curling.

4. The implant of claim 1, wherein the wall (31) is defined by a cylindrical base body and comprises extended end portions woven by monofilaments (37) and extending axially outwardly from both ends of the base body.

5. The implant of claim 1, wherein the wall (31) has a drug eluting layer, and the drugs in the drug eluting layer are the same as or different from the drugs in the biodegradable fiber bundles.

6. The implant of claim 1, wherein the wall has positioning portions extending radially outwardly at both ends of the wall.

7. The implant of claim 1, wherein the wall has at least one projection extending radially outwardly from the external surface of the wall.

## Patentansprüche

1. Implantat (30) zur Behandlung von Sinusitis oder allergischer Rhinitis, wobei das Implantat (30) eine sich in Umfangsrichtung erstreckende Wand (31) aufweist, die aus einem biologisch abbaubaren Polymer gebildet ist, und die Wand (31) eine Vielzahl von Öffnungen (32) umfasst, wobei in den Öffnungen (32) biologisch abbaubare Faserbündel angeordnet sind, die Arzneimittel enthalten
**gekennzeichnet durch**
dass biologisch abbaubare Faserbündel von dem Implantat (30) ausgehen und auf beiden Seiten des Implantats (30) verstreut sind,
wobei die Wand (31) **durch** eine in Umfangsrichtung geschlossene und an beiden Enden offene Rohrstruktur definiert ist, wobei die Wand gleichmäßig verteilte Öffnungen (32) mit in den Öffnungen (32) angeordneten Stützrippen (33) aufweist.

2. Implantat (60) zur Behandlung von Sinusitis oder allergischer Rhinitis, wobei das Implantat (60) eine sich in Umfangsrichtung erstreckende Wand (61) aufweist, die aus einem biologisch abbaubaren Polymer gebildet ist, und die Wand (61) eine Vielzahl von Öffnungen (62) umfasst, wobei in den Öffnungen (62) biologisch abbaubare Faserbündel angeordnet sind, die Arzneimittel enthalten,
**dadurch gekennzeichnet,**
**dass** biologisch abbaubare Faserbündel von dem Implantat (60) ausgehen und auf beiden Seiten des Implantats (60) verstreut sind,
wobei die Wand (61) in Umfangsrichtung nicht geschlossen ist und gleichmäßig verteilte Öffnungen (62) aufweist, wobei in den Öffnungen (62) biologisch abbaubare Fasern zur Bündelung angeordnet sind.

3. Implantat nach Anspruch 1, wobei die Wand (31) durch Weben von Monofilamenten, Schneiden von rohrförmigen Substraten oder Rollen von Blattsubstraten gebildet ist.

4. Implantat nach Anspruch 1, wobei die Wand (31) durch einen zylindrischen Grundkörper definiert ist und verlängerte Endabschnitte aufweist, die durch Monofilamente (37) gewebt sind und sich von beiden Enden des Grundkörpers axial nach außen erstrecken.

5. Implantat nach Anspruch 1, wobei die Wand (31) eine arzneimittelfreisetzende Schicht aufweist und die Arzneimittel in der arzneimittelfreisetzenden Schicht die gleichen oder andere sind als die Arzneimittel in den biologisch abbaubaren Faserbündeln.

6. Implantat nach Anspruch 1, wobei die Wand Positionierungsabschnitte aufweist, die sich an beiden Enden der Wand radial nach außen erstrecken.

7. Implantat nach Anspruch 1, wobei die Wand mindestens einen Vorsprung aufweist, der sich von der Außenfläche der Wand radial nach außen erstreckt.

## Revendications

1. Implant (30) pour traiter la sinusite ou la rhinite allergique, dans lequel l'implant (30) présente une paroi s'étendant circonférentiellement (31) formée d'un polymère biodégradable, et la paroi (31) inclut une pluralité d'ouvertures (32), avec des faisceaux de fibres biodégradables contenant des médicaments agencés dans les ouvertures (32),
**caractérisé en ce que**
les faisceaux de fibres biodégradables s'étendent à partir de l'implant (30) et sont diffusés sur les deux côtés de l'implant (30),
dans lequel la paroi (31) est définie par une structure tubulaire, qui est fermée dans la direction circonférentielle et qui est ouverte au niveau des deux extrémités, dans lequel la paroi présente des ouvertures réparties uniformément (32), avec des nervures de support (33) agencées dans les ouvertures (32).

2. Implant (60) pour traiter la sinusite ou la rhinite allergique, dans lequel l'implant (60) présente une paroi s'étendant circonférentiellement (61) formée d'un polymère biodégradable, et la paroi (61) inclut une pluralité d'ouvertures (62), avec des faisceaux de fibres biodégradables contenant des médicaments agencés dans les ouvertures (62),
**caractérisé en ce que**
les faisceaux de fibres biodégradables s'étendent à partir de l'implant (60) et sont diffusés sur les deux côtés de l'implant (60),
dans lequel la paroi (61) est non fermée dans la direction circonférentielle et présente des ouvertures réparties uniformément (62), avec des fibres biodégradables pour la formation de faisceaux agencées dans les ouvertures (62).

3. Implant selon la revendication 1, dans lequel la paroi (31) est formée par tissage de monofilaments, découpe de substrat tubulaire ou roulage de bord de substrat en feuille.

4. Implant selon la revendication 1, dans lequel la paroi (31) est définie par un corps de base cylindrique et comprend des parties d'extrémité étendues tissées par des monofilaments (37) et s'étendant axialement vers l'extérieur à partir des deux extrémités du corps de base.

5. Implant selon la revendication 1, dans lequel la paroi (31) présente une couche à élution de médicaments, et les médicaments dans la couche à élution de médicaments sont identiques aux médicaments dans les faisceaux de fibres biodégradables ou différents de ceux-ci.

6. Implant selon la revendication 1, dans lequel la paroi présente des parties de positionnement s'étendant radialement vers l'extérieur au niveau des deux extrémités de la paroi.

7. Implant selon la revendication 1, dans lequel la paroi présente au moins une saillie s'étendant radialement vers l'extérieur à partir de la surface externe de la paroi.
